# EUROPÄISCHE PATENTSCHRIFT

(11) **EP 1 400 211 B1**
(45) Veröffentlichungstag und Bekanntmachung des Hinweises auf die Patenterteilung: **08.06.2005**
(21) Anmeldenummer: 02020720.5
(22) Anmeldetag: 14.09.2002
(51) Int. Cl.: A61B 17/28

(54) **Endoskopisches Instrument mit abnehmbarem Werkzeugkopf**
Endoscopic intrument with detachable tool head
Instrument endoscopique avec tête d'outil amovible

(43) Veröffentlichungstag der Anmeldung: 24.03.2004
(73) Patentinhaber: Karl Storz GmbH & Co. KG, 78532 Tuttlingen (DE)
(72) Erfinder: Bacher, Uwe, 78532 Tuttlingen (DE)
(74) Vertreter: Hofmeister, Frank Horst

(56) Entgegenhaltungen:
- WO-A-02/26143
- WO-A-96/41574
- DE-U- 9 215 053
- US-A- 5 643 294

## Beschreibung

Die Erfindung betrifft ein medizinisches Instrument mit einem Schaft, einer am proximalen Ende des Schaftes angeordneten Handhabe sowie einem am distalen Ende des Schaftes angeordneten und über die Handhabe betätigbaren Werkzeug, wobei die Handhabe und das Werkzeug über mindestens eine Betätigungsstange in Wirkverbindung stehen und das Werkzeug über einen Werkzeugschaft lösbar an der Betätigungsstange festlegbar ist, wozu der Werkzeugschaft und die Betätigungsstange Vorsprünge und/oder Ausnehmungen aufweisen, die mit korrespondierenden Ausnehmungen bzw. Vorsprüngen des jeweils anderen Bauteils zumindest teilweise formschlüssig in Eingriff bringbar sind.

Gattungsgemäße medizinische Instrumente werden in der Praxis häufig als Greif-, Halte- und/oder Schneidwerkzeuge eingesetzt. So können die Maulteile Schneiden aufweisen, um Gewebe abzutrennen, oder stumpfe Flächen aufweisen, um beispielsweise abgetrenntes Gewebe zu halten, oder Blutgefäße abzuklemmen.

Um ein derartiges medizinisches Instrument einerseits möglichst flexibel verwenden zu können und andererseits die Reinigbarkeit des Instruments zu erleichtern, ist es aus der Praxis bekannt, das Werkzeug lösbar mit der Betätigungsstange zu verbinden. Die bekannten Verbindungen zwischen Werkzeug und Betätigungsstange weisen jedoch den Nachteil auf, dass sie, wie aus der EP 0 577 423 B1 bekannt, als Schraub- oder Rastverbindungen sehr aufwendig konstruiert sind, so dass ein einfacher, schneller und somit kostengünstiger Werkzeugwechsel bei diesen bekannten Instrumenten nicht möglich ist.

Ein gattungsgemäßes medizinischen Instrument ist beispielsweise aus der WO-A-9641574 bekannt. Bei diesem bekannten medizinischen Instrument ist die Betätigungsstange über Vorsprünge und/oder Ausnehmungen mit korrespondierenden Vorsprünge und/oder Ausnehmungen des Werkzeugschaftes formschlüssig in Eingriff bringbar. Die formschlüssige Kopplung der Bauteile erfolgt bei diesem bekannten Instrument dadurch, dass das distale Ende der Betätigungsstange hammerkopfförmig ausgebildet ist und im Werkzeugschaft eine korrespondierend geformte Durchgangsöffnung zur Aufnahme dieses Hammerkopfes ausgebildet ist.

Dieses bekannte medizinische Instrument weist den konstruktiven Nachteil auf, dass die im Werkzeugschaft ausgebildete Ausnehmung zur Aufnahme des hammerkopfförmigen Vorsprungs der Betätigungsstange als Durchgangsöffnung ausgebildet ist, weshalb bei der Montage darauf zu achten ist, dass die in den Werkzeugschaft eingesetzte Betätigungsstange nicht auf der anderen Seite wieder aus der Durchgangsöffnung herausfällt.

Ein weiteres medizinisches Instrument ist aus der US 5 496 347 bekannt. Bei diesem bekannten Instrument ist die Betätigungsstange direkt an den Maulteilen des Werkzeugs festgelegt. Zu diesem Zweck weist das distale Ende der Betätigungsstange Ausnehmungen auf, in die an den Maulteilen ausgebildete Nocken eingreifen. Um die Verbindung der Betätigungsstange mit den Maulteilen in radialer Richtung des Schaftes zu fixieren, ist einerseits die Betätigungsstange zwischen den beiden Maulteilen angeordnet und andererseits das distale Ende des Schaftes so ausgebildet, das die Betätigungsstange und die Maulteile in diesem Kopplungsbereich von einer Hülse umgeben sind, die innerhalb des hohlen Schaftes angeordnet ist und zusätzlich als Führung für die Betätigungsstange dient.

Diese bekannte Konstruktion ermöglicht zwar das Festlegen des Werkzeugs an der Betätigungsstange ohne Schraubverbindungen, jedoch ist der Montageaufwand, insbesondere durch die Verwendung der zusätzlichen Hülse sowie das Verbinden der Betätigungsstange mit den einzelnen Maulteilen des Werkzeugs, so groß, dass ebenfalls ein einfacher, schneller und somit kostengünstiger Werkzeugwechsel bei diesem Instrument nicht möglich ist.

Davon ausgehend liegt der Erfindung die **Aufgabe** zugrunde, ein medizinisches Instrument der eingangs genannten Art so auszugestalten, dass das Werkzeug insbesondere als Einweg-Werkzeug einfach und schnell an der Betätigungsstange festlegbar ist.

Die **Lösung** dieser Aufgabenstellung ist erfindungsgemäß dadurch gekennzeichnet, dass die miteinander korrespondierenden Ausnehmungen und Vorsprüngen so ausgebildet sind, dass das Werkzeug und die Betätigungsstange über eine Bewegung in ausschließlich einer Richtung im wesentlichen senkrecht zur Längsachse der Betätigungsstange miteinander in Eingriff bringbar sind und die miteinander gekoppelten Bauteile in die anderen Richtungen gegeneinander fixiert sind.

Aufgrund der erfindungsgemäßen Ausgestaltung der Kupplungsbereiche von Werkzeug und Betätigungsstange ist es erstmalig möglich, die miteinander zu verbindenden Bauteile ohne zusätzliche Sicherungselemente im wesentlichen durch eine Steckverbindung aneinander zu fixieren. Der Kopplungsmechanismus zwischen der Betätigungsstange einerseits und dem Werkzeugschaft andererseits ist dabei so ausgebildet ist, dass das jeweils aufzunehmende Bauteil von dem anderen Bauteil haltend ergriffen wird und so ein Durchfallen und/oder wieder Auseinanderfallen der Bauteile verhindert wird, da die Bauteile ausschließlich in einer Richtung, nämlich der entgegen der Einsetzrichtung, trennbar sind. Das bloße senkrechte Ineinandersetzen der Bauteile ergibt somit schon ein mehr oder weniger funktionstüchtiges Instrument; auf jeden Fall aber wird die Montage des Instruments deutlich erleichtert, da sich die Bauteile gegenseitig fixieren. Um das erfindungsgemäße medizinische Instrument möglichst vielseitig verwenden zu können, wird gemäß einer bevorzugten Ausführungsform vorgeschlagen, dass das Werkzeug derart an der Betätigungsstange festlegbar ist, dass Kräfte in Längsrichtung der Betätigungsstange und/oder Torsionskräfte auf das Werkzeug übertragbar sind.

Das Verbinden von Werkzeug und Betätigungsstange erfolgt vorzugsweise über eine Bewegung im wesentlichen senkrecht zur Längsachse der Betätigungsstange, wobei diese Bewegung als Steckbewegung in Richtung senkrecht zur Längsachse der Betätigungsstange oder als Schwenkbewegung um eine Achse senkrecht zur Längsachse der Betätigungsstange ausführbar ist.

Weiterhin wird mit der Erfindung vorgeschlagen, dass das Werkzeug lösbar am Werkzeugschaft festlegbar ist. Bei dieser Ausgestaltungsform kann der Werkzeugschaft beispielsweise als Adapter verwendet werden, um unterschiedliche Werkzeuge mit dem bezüglich der Betätigungsstangenverbindung identischen Adapter mit der Betätigungsstange in Eingriff zu bringen.

Gemäß einer bevorzugten Ausführungsform der Erfindung sind die Betätigungsstange sowie der Werkzeugschaft im Querschnitt im wesentlich kreisförmig ausgebildet. Diese Ausgestaltungsform ist besonders vorteilhaft, wenn Torsionskräfte auf das Werkzeug übertragen werden sollen.

Gemäß einer ersten praktischen Ausführungsform mit im Querschnitt im wesentlichen kreisförmiger Ausbildung von Betätigungsstange und Werkzeugschaft ist im Bereich des distalen Endes der runden Betätigungsstange zumindest einseitig eine tangentiale Abflachung an der Betätigungsstange derart ausgebildet, dass das distale Ende der Betätigungsstange weiterhin einen die Abflachung in radialer Richtung überragenden Kopfbereich aufweist. Der proximale Bereich des Werkzeugschaftes weist hierbei eine Hinterschneidung zur Aufnahme des Kopfbereichs der Betätigungsstange sowie eine mit der tangentialen Abflachung der Betätigungsstange korrespondierende Aufnahme auf.

Während es bei der vorgenannten Ausführungsform möglich ist, die Abflachung asymmetrisch anzuordnen, wird gemäß einer zweiten alternativen Ausgestaltungsform vorgeschlagen, dass die tangentiale Abflachung der Betätigungsstange als von zwei einander gegenüberliegenden Seiten abgeflachter Mittelsteg und die korrespondierende Aufnahme am Werkzeugschaft als radialer Schlitz ausgebildet sind.

Weiterhin wird mit der Erfindung vorgeschlagen, dass die Betätigungsstange und das Werkzeug über mindestens einen quer zur Instrumentenlängsachse verlaufenden Steg miteinander koppelbar sind, wobei der Steg entweder an der Betätigungsstange oder am Werkzeugschaft ausgebildet ist und in eine entsprechende Ausnehmung im Werkzeugschaft oder in der Betätigungsstange eingreift.

Die Verwendung des Stegs zum Verbinden der Betätigungsstange mit dem Werkzeug kann dabei zusätzlich oder alternativ zu der Ausbildung der Hinterschneidung und des Kopfbereichs vorgesehen sein.

Schließlich wird mit der Erfindung vorgeschlagen, dass zur Übertragung von Zugoder Druckkräften im Kupplungsbereich zwischen der Betätigungsstange und dem Werkzeug ein Federelement angeordnet ist. Neben der Möglichkeit, die Federkraft des Federelements zu nutzen, um beispielsweise bei Aufbringen einer Druckkraft das Werkzeug zu öffnen, unterstützt die Federkraft die formschlüssige Fixierung der Bauteile Betätigungsstange und Werkzeugschaft.

Weitere Merkmale und Vorteile der Erfindung ergeben sich anhand der nachfolgenden Beschreibung der zugehörigen Zeichnung, in der drei Ausführungsbeispiele eines erfindungsgemäßen medizinischen Instruments beispielhaft schematisch dargestellt sind. In der Zeichnung zeigt:
- Fig. 1: eine schematische Seitenansicht eines erfindungsgemäßen medizinischen Instruments;
- Fig. 2a: eine perspektivische Seitenansicht einer Betätigungsstange und eines Werkzeugs gemäß einer ersten erfindungsgemäßen Ausführungsform im zusammengesetzten Zustand;
- Fig. 2b: eine Ansicht gemäß Fig. 2a, jedoch die Betätigungsstange und das Werkzeug im voneinander getrennten Zustand darstellend;
- Fig. 3: eine Ansicht gemäß Fig. 2b, eine zweite erfindungsgemäße Ausführungsform darstellend und
- Fig. 4: eine Ansicht gemäß Fig. 2b, eine dritte erfindungsgemäße Ausführungsform darstellend.

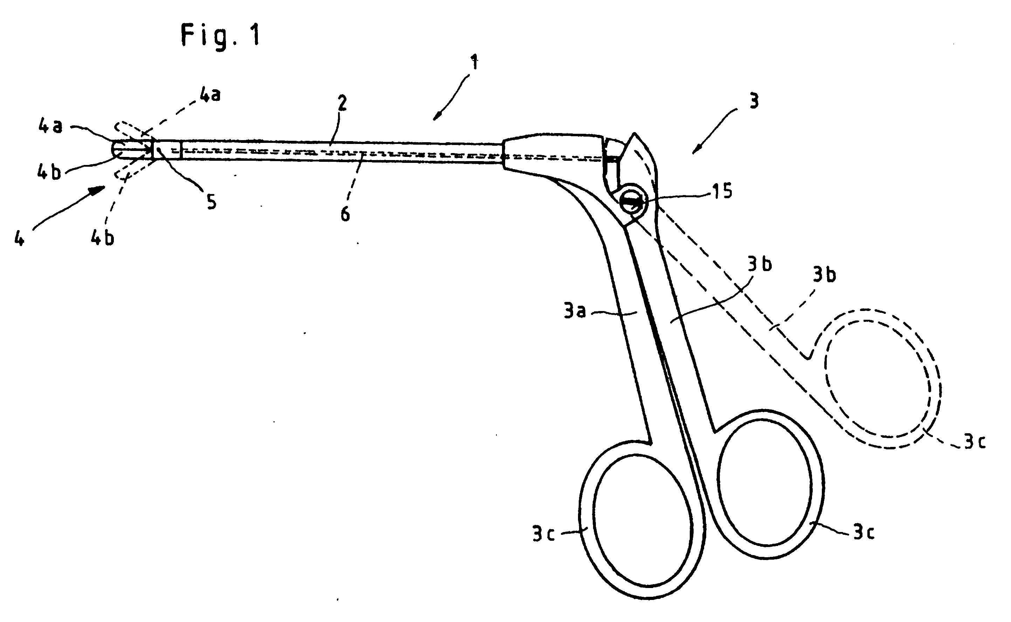

Die Abbildung Fig. 1 zeigt eine Seitenansicht eines medizinischen Instruments 1, dessen Kraftübertragungsmechanismus vielseitig verwendet werden kann, wie beispielsweise für Stanzen, Scheren, Nadelhalter, Faßinstrumente und dergleichen.

Das dargestellte medizinische Instrument 1 besteht im wesentlichen aus einem hohlen Schaft 2, an dessen proximalem Ende eine Handhabe 3 angeordnet ist, die aus einem starren Griffteil 3a und einem gegenüber dem starren Griffteil 3a verschwenkbaren Griffteil 3b besteht. Am distalen Ende des Schaftes 2 ist ein Werkzeug 4 angeordnet, welches von zwei gegeneinander um einen gemeinsamen Drehpunkt 5 verschwenkbaren Maulteilen 4a und 4b gebildet wird.

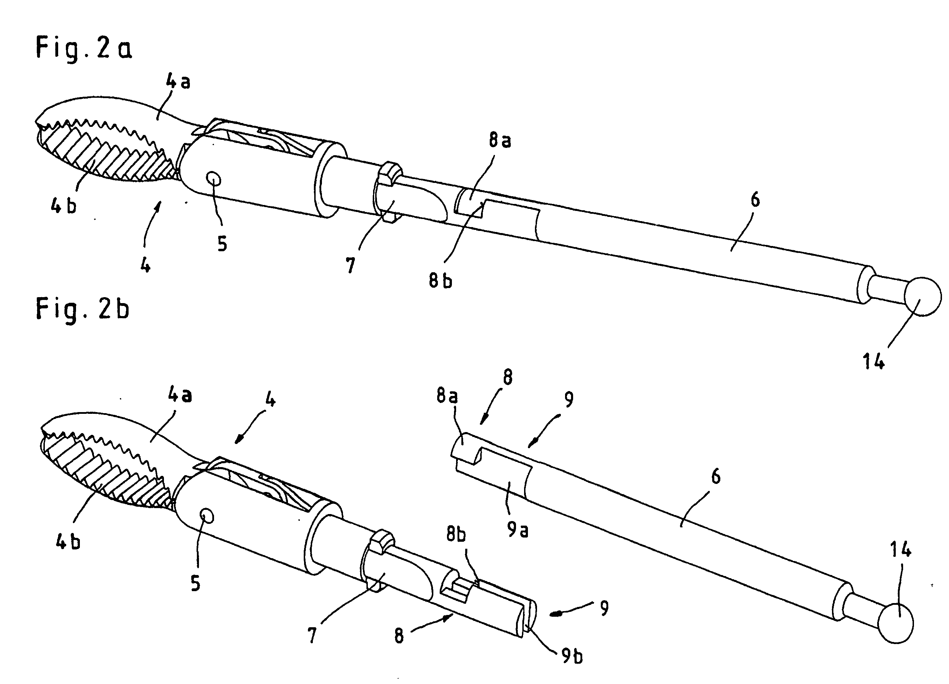

Wie aus den Ansichten gemäß Fig. 2a bis 4 in Zusammenschau mit der Komplettansicht gemäß Fig. 1 ersichtlich, sind die Maulteile 4a und 4b des Werkzeugs 4 und das verschwenkbare Griffteil 3b der Handhabe 3 über eine Betätigungsstange 6 miteinander so verbunden, daß durch das Verstellen des Griffteils 3b die Maulteile 4a und 4b von der geschlossenen Stellung (durchgezogene Darstellung in Fig. 1) in die geöffnete Stellung (gestrichelte Darstellung in Fig. 1) bzw. umgekehrt überführbar sind. Die jeweils zugehörige Stellung des verschwenkbaren Griffteils 3b ist in der Abbildung Fig.1 ebenfalls durchgezogen (für die geschlossene Stellung) und gestrichelt (für die geöffnete Stellung) dargestellt.

Den Darstellungen gemäß Fig. 2a bis Fig. 4 ist weiterhin zu entnehmen, dass die Betätigungsstange 6 nicht unmittelbar am Werkzeug 4 festgelegt ist, sondern an einem Werkzeugschaft 7 festgelegt ist, der bei den dargestellten Ausführungsbeispielen lösbar mit dem Werkzeug 4 verbunden ist und in direkter Wirkverbindung mit den Maulteilen 4a und 4b des Werkzeugs 4 steht.

Zum Verbinden der Betätigungsstange 6 mit dem Werkzeugschaft 7 sind bei dargestellten Ausführungsbeispielen sowohl an der Betätigungsstange 6 als auch am Werkzeugschaft 7 Vorsprünge 8 und Ausnehmungen 9 ausgebildet, die mit entsprechenden Vorsprünge 8 und Ausnehmungen 9 des jeweils anderen Bauteils (6, 7 bzw. 7, 6) derart korrespondieren, dass diese formschlüssig miteinander in Eingriff bringbar sind. Bei den dargestellten Ausführungsbeispielen ist diese einander entsprechende Ausgestaltung der Vorsprünge 8 und Ausnehmungen 9 insbesondere der Abbildung Fig. 2a zu entnehmen, die einen im zusammengesetzten Zustand durchgehend runden Querschnitt von Betätigungsstange 6 und Werkzeugschaft 7 zeigt. Selbstverständlich können die Betätigungsstange 6 und der Werkzeugschaft 7 auch unrunde und/oder von einander abweichende Querschnittsformen aufweisen.

Bei den in den Abbildungen Fig. 2a bis Fig. 4 dargestellten Ausführungsformen werden die Ausnehmungen 9 an der Betätigungsstange 6 durch tangentiale Abflachungen gebildet, die von zwei einander gegenüberliegenden Seiten an der Betätigungsstange 6 so ausgebildet sind, dass die Betätigungsstange im Bereich dieser Abflachung nur noch aus einem schmalen Mittelsteg 9a besteht. Das distale Ende der Betätigungsstange 6 bildet ein den Mittelsteg 9a in radialer Richtung überragender, einen Vorsprung 8 bildender Kopfbereich 8a.

Zur Aufnahme des Mittelstegs 9a sowie des Kopfbereichs 8a der Betätigungsstange 6 sind am Werkzeugschaft 7 als Ausnehmung 9 ein radialer Schlitz 9b sowie als Vorsprung 8 eine Hinterschneidung 8b ausgebildet.

Wie aus Fig. 2a ersichtlich, fügen sich die jeweiligen Vorsprünge 8 und Ausnehmungen 9 von Betätigungsstange 6 und Werkzeugschaft 7 im zusammengesetzten Zustand so zusammen, dass diese formschlüssig ineinander greifen.

Alternativ zu den dargestellten Ausführungsformen, bei denen die Ausnehmung 9 an der Betätigungsstange 6 als den Mittelsteg 9a ausbildende symmetrische zweiseitige Abflachung der Betätigungsstange 6 ausgebildet ist, ist es selbstverständlich auch möglich, die Abflachung asymmetrisch nur einseitig oder unterschiedlich stark ausgebildet zweiseitig auszuführen. Der Mittelsteg 9a kann dabei, wie dargestellt, quaderförmig ausgebildet sein, aber beispielsweise ebenso einen sich in Einsteckrichtung verjüngenden Querschnitt aufweisen. Auch ist es möglich, dass sich der Querschnitt des Mittelstegs 9a in Axialrichtung ändert.

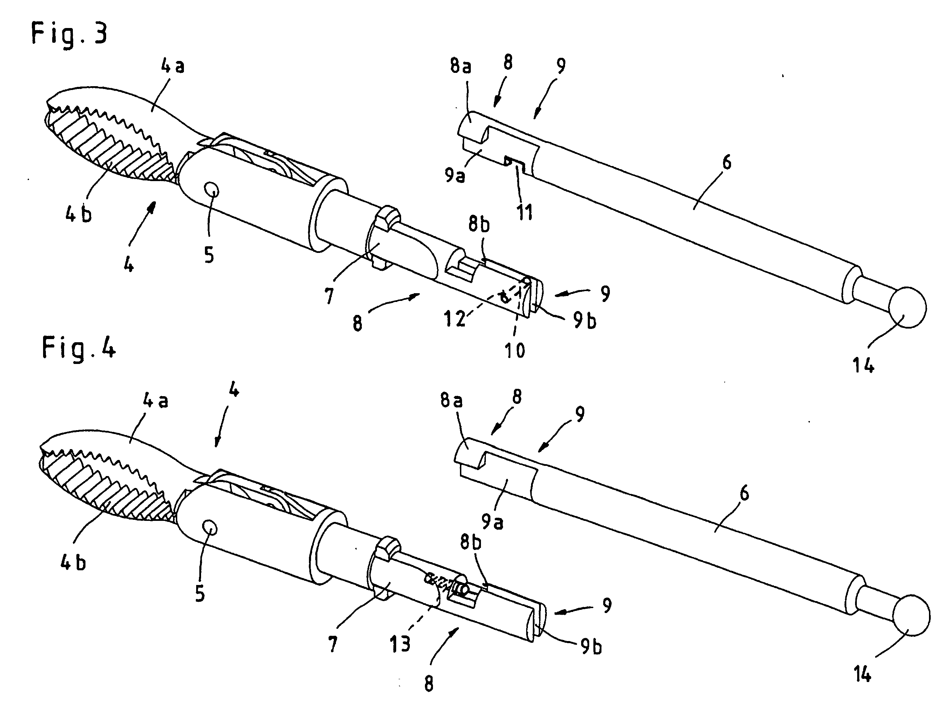

Die in Fig. 3 dargestellte zweite Ausführungsform unterscheidet sich von der gemäß Fig. 2b dadurch, dass im Werkzeugschaft 7 zusätzlich ein quer zur Instrumentenlängsachse verlaufender Steg 10 angeordnet ist, der in eine entsprechende Ausnehmung 11 in der Betätigungsstange 6 eingreift. Bei der gezeigten Ausführungsform ist der Steg 10 in einer im Werkzeugschaft 7 ausgebildeten Bohrung 12 gelagert. Alternativ besteht die Möglichkeit, den Steg 10 an der Betätigungsstange 6 anzuordnen und die korrespondierende Ausnehmung 11 am Werkzeugschaft 7 auszubilden, wobei es in beiden Fällen auch möglich ist, den Steg 10 einstückig mit der Betätigungsstange 6 oder dem Werkzeugschaft 7 auszubilden.

Die in Fig. 4 dargestellte dritte Ausführungsform unterscheidet sich von der gemäß Fig. 2b dadurch, dass im Kupplungsbereich zwischen der Betätigungsstange 6 und dem Werkzeug 4 ein Federelement 13 angeordnet ist. Das nur beispielhaft schematisch eingezeichnete Federelement 13 kann dazu dienen, beispielsweise bei Aufbringen einer Druckkraft das Werkzeug 4 gefedert zu öffnen, oder aber die formschlüssige Fixierung der Bauteile Betätigungsstange 6 und Werkzeugschaft 7 zu unterstützen. Wenn das Federelement 13 zwischen dem Werkzeug 4 und dem Werkzeugschaft 7 angeordnet ist, kann das Federelement 13 eine Betätigung des Werkzeugs 4 allein in nur eine Richtung (Öffnen oder Schließen) bewirken. Die entsprechende andere Bewegung (Schließen oder Öffnen) ist dann durch die Betätigung der Betätigungsstange 6 zu realisieren.

Insbesondere bei der Ausgestaltung des Kupplungsbereichs zwischen der Betätigungsstange 6 und dem Werkzeug 4 mit einem Federelement 13 ist es möglich, auf den in den Abbildungen Fig. 2a bis Fig. 4 dargestellten Kopfbereich 8a an der Betätigungsstange 6 zu verzichten.

In diesem Fall können über die Betätigungsstange 6 keine Zugkräfte, sondern nur Druck- und Torsionskräfte auf das Werkzeug 4 übertragen werden. Die Zugkräfte können bei dieser Ausgestaltungsform durch das Federelement 13 übernommen werden, dessen Federkraft das Werkzeug 4 schließt bzw. dessen Federkraft zum Öffnen des Werkzeugs 4 durch die über die Betätigungsstange 6 aufgebrachte Druckkraft überwunden werden muß.

Selbstverständlich sind auch konstruktive Lösungen möglich, bei denen über die Betätigungsstange 6 eine Zugkraft aufgebracht wird und die Druckkraft durch die Federkraft des Federelements 13 bewirkt wird.

### Das Betätigen des medizinischen Instruments 1 geschieht wie folgt:

Zunächst werden ausgehend von der in Fig. 2b dargestellten getrennten Ausgangsstellung die Betätigungsstange 6 und der Werkzeugschaft 7 über eine Bewegung im wesentlichen senkrecht zur Längsachse der Betätigungsstange 6 durch Einpassen der gegenseitigen Vorsprünge 8 und Ausnehmungen 9 so miteinander in Eingriff gebracht, dass sich die in Fig. 2a dargestellte zusammengesetzte Ausgestaltung ergibt.

Anschließend wird die Betätigungsstange 6 mit dem proximalen Ende voran in den hohlen Schaft 2 eingeführt. Zum Verbinden der Betätigungsstange 6 mit dem verschwenkbaren Griffteil 3b der Handhabe 3 ist am proximalen Ende der Betätigungsstange 6 eine Gelenkkugel 14 angeordnet, die in eine entsprechende, nicht dargestellte, Kugelaufnahme am Griffteil 3b einsetzbar ist.

Zum sicheren Ergreifen der Griffteile 3a, 3b der Handhabe 3 weisen diese an ihren freien Enden Fingerösen 3c auf. Beim dargestellten Ausführungsbeispiel ist das Griffteil 3b um eine Schwenkachse 15 gegenüber dem anderen, starren Griffteil 3a verschwenkbar.

Durch die Kopplung des verschwenkbaren Griffteils 3b über die Betätigungsstange 6 mit dem Werkzeug 4 lassen sich die Maulteile 4a, 4b des Werkzeugs 4 öffnen und schließen.

Aufgrund der einfachen Kopplung der Betätigungsstange 6 am Werkzeug 4 bzw. dem Werkzeugschaft 7 durch die Ausbildung der Vorsprünge 8 und Ausnehmungen 9 lassen sich besonders einfach und schnell neue Werkzeuge 4 an der Betätigungsstange 6 festlegen, wodurch insbesondere der Einsatz vor Einmal-Werkzeugen erleichtert wird.

Die Kopplung der Betätigungsstange 6 am Werkzeug 4 bzw. dem Werkzeugschaft 7 ist dabei ferner so ausgebildet, dass sowohl Kräfte in Längsrichtung der Betätigungsstange 6 als auch Torsionskräfte auf das Werkzeug 4 übertragbar sind.

### Bezugszeichenliste

- 1: medizinisches Instrument
- 2: Schaft
- 3: Handhabe
- 3a: starres Griffteil
- 3b: verschwenkbares Griffteil
- 3c: Fingeröse
- 4: Werkzeug
- 4a: Maulteil
- 4b: Maulteil
- 5: Drehpunkt
- 6: Betätigungsstange
- 7: Werkzeugschaft
- 8: Vorsprung
- 8a: Kopfbereich
- 8b: Hinterschneidung
- 9: Ausnehmung
- 9a: Mittelsteg
- 9b: Schlitz
- 10: Steg
- 11: Ausnehmung
- 12: Bohrung
- 13: Federelement
- 14: Gelenkkugel
- 15: Schwenkachse

## Patentansprüche

1. Medizinisches Instrument mit einem Schaft (2), einer am proximalen Ende des Schaftes (2) angeordneten Handhabe (3) sowie einem am distalen Ende des Schaftes (2) angeordneten und über die Handhabe (3) betätigbaren Werkzeug (4), wobei die Handhabe (3) und das Werkzeug (4) über mindestens eine Betätigungsstange (6) in Wirkverbindung stehen und das Werkzeug (4) über einen Werkzeugschaft (7) lösbar an der Betätigungsstange (6) festlegbar ist, wozu der Werkzeugschaft (7) und die Betätigungsstange (6) Vorsprünge (8) und/oder Ausnehmungen (9) aufweisen, die mit korrespondierenden Ausnehmungen (9) bzw. Vorsprüngen (8) des jeweils anderen Bauteils (6, 7 bzw. 7, 6) zumindest teilweise formschlüssig in Eingriff bringbar sind,
**dadurch gekennzeichnet,**
**dass** die miteinander korrespondierenden Ausnehmungen (9) und Vorsprüngen (8) so ausgebildet sind, dass das Werkzeug (4) und die Betätigungsstange (6) über eine Bewegung in ausschließlich einer Richtung im wesentlichen senkrecht zur Längsachse der Betätigungsstange (6) miteinander in Eingriff bringbar sind und die miteinander gekoppelten Bauteile (6 und 7) in die anderen Richtungen gegeneinander fixiert sind.

2. Medizinisches Instrument nach Anspruch 1, **dadurch gekennzeichnet, dass** die Betätigungsstange (6) sowie der Werkzeugschaft (7) im Querschnitt im wesentlich kreisförmig ausgebildet sind.

3. Medizinisches Instrument nach Anspruch 2, **dadurch gekennzeichnet, dass** im Bereich des distalen Endes der runden Betätigungsstange (6) zumindest einseitig ein tangentiale Abflachung an der Betätigungsstange (6) derart ausgebildet ist, dass das distale Ende der Betätigungsstange (6) weiterhin einen die Abflachung in radialer Richtung überragenden Kopfbereich (8a) aufweist, und dass der proximale Bereich des Werkzeugschaftes (7) eine Hinterschneidung (8b) zur Aufnahme des Kopfbereichs (8a) der Betätigungsstange (6) sowie eine mit der tangentialen Abflachung der Betätigungsstange (6) korrespondierende Aufnahme aufweist.

4. Medizinisches Instrument nach Anspruch 3, **dadurch gekennzeichnet, dass** die tangentiale Abflachung der Betätigungsstange (6) als von zwei einander gegenüberliegenden Seiten abgeflachter Mittelsteg (9a) und die korrespondierende Aufnahme am Werkzeugschaft (7) als radialer Schlitz (9b) ausgebildet sind.

5. Medizinisches Instrument nach mindestens einem der Ansprüche 1 bis 4, **dadurch gekennzeichnet, dass** das Werkzeug (4) derart an der Betätigungsstange (6) festlegbar ist, dass Kräfte in Längsrichtung der Betätigungsstange (6) und/oder Torsionskräfte auf das Werkzeug (4) übertragbar sind.

6. Medizinisches Instrument nach mindestens einem der Ansprüche 1 bis 5, **dadurch gekennzeichnet, dass** das Werkzeug (4) lösbar am Werkzeugschaft (7) festlegbar ist.

7. Medizinisches Instrument nach mindestens einem der Ansprüche 1 bis 6, **dadurch gekennzeichnet, dass** die Betätigungsstange (6) und das Werkzeug (4) über mindestens einen quer zur Instrumentenlängsachse verlaufenden Steg (10) miteinander koppelbar sind, wobei der Steg (10) einerseits in einer Bohrung (12) in der Betätigungsstange (6) oder im Werkzeugschaft (7) gelagert ist und andererseits in eine entsprechende Ausnehmung (11) im Werkzeugschaft (7) oder in der Betätigungsstange (6) eingreift.

8. Medizinisches Instrument nach mindestens einem der Ansprüche 1 bis 7, **dadurch gekennzeichnet, dass** zur Übertragung von Zug- oder Druckkräften im Kupplungsbereich zwischen der Betätigungsstange (6) und dem Werkzeug (4) ein Federelement (13) angeordnet ist.

9. Medizinisches Instrument nach Anspruch 8, **dadurch gekennzeichnet, dass** das Werkzeug (4) über das Federelement (13) betätigbar ist.

## Claims

1. Medical instrument with a shaft (2), a handle (3) arranged at the proximal end of the shaft (2), and a tool (4) arranged at the distal end of the shaft (2) and activated via the handle (3), said handle (3) and said tool (4) being operatively connected via at least one activation rod (6), and said tool (4) being able to be secured releasably on the activation rod (6) via a tool shaft (7), for which purpose the tool shaft (7) and the activation rod (6) have projections (8) and/or recesses (9) which can be brought at least partially into form-fit engagement with corresponding recesses (9) and projections (8) of the respective other component (6, 7 or 7, 6), **characterized in that** the recesses (9) and projections (8) corresponding with one another are configured in such a way that the tool (4) and the activation rod (6) can be brought into engagement with one another by a movement exclusively in a direction substantially perpendicular to the longitudinal axis of the activation rod (6), and the components (6 and 7) coupled to one another are fixed relative to one another in the other directions.

2. Medical instrument according to Claim 1,
**characterized in that** the activation rod (6) and the tool shaft (7) are substantially circular in cross section.

3. Medical instrument according to Claim 2,
**characterized in that**, in the area of the distal end of the round activation rod (6), a tangential flattened surface is formed on at least one side of the activation rod (6) in such a way that the distal end of the activation rod (6) also has a head area (8a) protruding radially over the flattened surface, and **in that** the proximal area of the tool shaft (7) has an undercut (8b) for receiving the head area (8a) of the activation rod (6), and a recess corresponding with the tangential flattened surface of the activation rod (6).

4. Medical instrument according to Claim 3,
**characterized in that** the tangential flattened surface of the activation rod (6) is configured as a central bridge (9a) flattened from two opposite sides, and the corresponding recess on the tool shaft (7) is configured as a radial slit (9b).

5. Medical instrument according to at least one of Claims 1 to 4, **characterized in that** the tool (4) can be secured on the activation rod (6) in such a way that forces acting in the longitudinal direction of the activation rod (6) and/or torsional forces can be transmitted to the tool (4).

6. Medical instrument according to at least one of Claims 1 to 5, **characterized in that** the tool (4) can be secured releasably on the tool shaft (7).

7. Medical instrument according to at least one of Claims 1 to 6, **characterized in that** the activation rod (6) and the tool (4) can be coupled to one another via at least one bridge (10) extending transversely with respect to the longitudinal axis of the instrument, which bridge (10), on the one hand, is mounted in a bore (12) in the activation rod (6) or in the tool shaft (7) and, on the other hand, engages in a corresponding recess (11) in the tool shaft (7) or in the activation rod (6).

8. Medical instrument according to at least one of Claims 1 to 7, **characterized in that**, in order to transmit pulling or pushing forces, a spring element (13) is arranged in the coupling area between the activation rod (6) and the tool (4).

9. Medical instrument according to Claim 8,
**characterized in that** the tool (4) can be activated via the spring element (13).

## Revendications

1. Instrument médical, avec une tige (2), avec une manette (3) disposée sur l'extrémité proximale de la tige (2) et avec un outil (4) disposé sur l'extrémité distale de la tige (2) et actionnable par l'intermédiaire de la manette (3), la manette (3) et l'outil (4) étant en interaction par l'intermédiaire d'au moins une barre de manipulation (6) et l'outil (4) pouvant être fixé de façon amovible sur la barre de manipulation (6), par l'intermédiaire d'une tige d'outil (7), la tige d'outil (7) et la barre de manipulation (6) présentant à cet effet des saillies (8) et/ou des creux (9), qui peuvent s'accrocher au moins partiellement par complémentarité de forme dans des saillies (9) correspondantes ou dans des creux (8) correspondants de l'autre élément respectif (6, 7 ou 7, 6),
**caractérisé en ce que** les creux (9) et les saillies (8) correspondants entre eux sont conçus de façon à ce que l'outil (4) et la barre de manipulation (6) puissent être accrochés l'un dans l'autres par un mouvement dans une seule direction, sensiblement perpendiculaire à l'axe longitudinal de la barre de manipulation (6) et **en ce que** les éléments accouplés l'un à l'autre (6 et 7) sont fixés l'un contre l'autre dans les autres directions.

2. Instrument médical selon la revendication 1,
**caractérisé en ce que** la barre de manipulation (6), ainsi que la tige d'outil (7) sont conçues avec une section transversale sensiblement circulaire.

3. Instrument médical selon la revendication 2,
**caractérisé en ce que** dans la région de l'extrémité distale de la barre de manipulation ronde (6), un méplat tangentiel est formé au moins sur un côté de la barre de manipulation (6), de façon à ce que l'extrémité distale de la barre de manipulation (6) soit munie par ailleurs d'une région de tête (8a), saillant par dessus le méplat en direction axiale et **en ce que** la région proximale de la tige d'outil (7) est munie d'une contre dépouille (8b), pour loger la région de tête (8a) de la barre de manipulation (6), ainsi que d'un creux correspondant avec le méplat tangentiel de la barre de manipulation (6).

4. Instrument médical selon la revendication 3,
**caractérisé en ce que** le méplat tangentiel de la barre de manipulation (6) est conçu sous la forme d'une barrette centrale (9a) formée de deux faces opposées aplaties et **en ce que** le creux correspondant sur la tige d'outil (7) est conçu en tant que fente radiale (9b).

5. Instrument médical selon au moins l'une quelconque des revendications 1 à 4, **caractérisé en ce que** l'outil (4) peut être fixé sur la barre de manipulation (6) de façon à ce que des forces soient transmissibles en direction longitudinale de la barre de manipulation (6) et/ou des forces de torsion soient transmissibles sur l'outil (4).

6. Instrument médical selon au moins l'une quelconque des revendications 1 à 5, **caractérisé en ce que** l'outil (4) peut être fixé de façon amovible sur la tige d'outil (7).

7. Instrument médical selon au moins l'une quelconque des revendications 1 à 6, **caractérisé en ce que** la barre de manipulation (6) et l'outil (4) peuvent être accouplés par l'intermédiaire d'au moins une barrette (10) s'étendant à la transversale de l'axe de l'instrument, la barrette (10) étant logée d'une part dans un alésage (12) dans la barre de manipulation (6) ou dans la tige de l'outil (7) et s'accrochant d'autre part dans un creux (11) correspondant dans la tige de l'outil (7) ou dans la barre de manipulation (6).

8. Instrument médical selon au moins l'une quelconque des revendications 1 à 7, **caractérisé en ce que** pour la transmission de forces de traction ou de pression, un élément à ressort (13) est disposé dans la région d'accouplement entre la barre de manipulation (6) et l'outil (4).

9. Instrument médical selon la revendication 8,
**caractérisé en ce que** l'outil (4) peut être actionné par l'intermédiaire de l'élément à ressort (13).
